# EUROPEAN PATENT APPLICATION

(11) **EP 4 316 431 A1**
(43) Date of publication of application: **07.02.2024**
(21) Application number: 23189040.1
(22) Date of filing: 01.08.2023
(51) Int. Cl.: A61F 5/44, A61F 5/441, A61F 5/453

(54) **MALE URINARY INCONTINENCE DEVICE**

(30) Priority: 03.08.2022 IT 202200016563
(71) Applicant: Dindelli, Moreno, 20054 Segrate (IT)
(72) Inventor: Dindelli, Moreno, 20054 Segrate (IT)
(74) Representative: Colombo, Stefano Paolo

(57) **Abstract**

A device (1) for male urinary incontinence, comprising a first collection bag (2) of a user's urine having a rear wall (3), a front wall (4), an inner surface (5), and an outer surface (6), said first bag (2) being provided with a through hole (7) for introducing said user's genitals, characterized in that said device (1) further comprises a second urine-permeable bag (9), placed inside said first bag (2), in which a hydrogel-based product adapted to transform said user's urine from the liquid to the solid state is housed.

## Description

### Field of application

The present invention relates to a device for urinary incontinence, in particular male urinary incontinence.

### Background art

Urinary incontinence is normally understood as an individual's inability to retain urine due to anatomical or functional alterations of the bladder sphincters.

The male population in particular, as it ages, encounters various disorders in the urological field, including urinary urge incontinence. Such a condition is due to the natural aging of the urinary tract, but especially to the frequent prostate and bladder diseases afflicting men and the consequences of surgical therapies and/or radiotherapies therefor.

There are several remedies on the market, both in terms of drugs and medical devices, which seek to combat male urinary incontinence, although such remedies often prove to be poorly effective and inappropriate.

The following known remedies are used to manage urinary incontinence:
- Diapers and covers: these are absorbent devices which, depending on the case, can be worn directly in place of normal briefs. Once they are filled with urine, they are foul-smelling and also require a frequent change because they easily macerate skin, causing skin abrasions up to causing real ulcers. They are also highly polluting and not very environmentally-sustainable products.
- Intravesical urinary catheters: these are invasive devices which are not directly manageable by the patient or his family members and are often the vehicle for frequent urinary tract infections.
- External urinary catheters: these are sheaths made of latex or synthetic material provided with a discharge tube and connected to an external collection bag. They are a good alternative to internal catheterization. These devices can also cause skin maceration or irritation as well, and for this reason it is important for them to be replaced daily.
- Medications: few exist, and above all they are poorly effective and potentially harmful.
- Surgical devices: such devices are always invasive and, to date, poorly effective.

Therefore, it is the technical task of the present invention to manufacture a male urinary incontinence device which allows eliminating the technical drawbacks complained of by the prior art. Within the scope of this technical task, it is an object of the invention to provide a male urinary incontinence device specifically designed to respond to the different incontinence needs and which takes into account the user's social life and normal daily activities.

It is another object of the present invention to provide a male urinary incontinence device which is practical to both use and change and which respects the morphology of male genitals.

It is another object of the present invention to provide a male urinary incontinence device which is easy to use and inexpensive.

It is a not last object of the present invention to provide a male urinary incontinence device which is environmentally sustainable. In order to obviate the drawbacks of the prior art and to achieve these and further objects and advantages, the Applicant has studied and implemented the present invention.

### Summary of the invention

The technical task, as well as these and other objects, according to the present invention, are achieved by a male urinary incontinence device comprising a first collection bag of a user's urine having a rear wall, a front wall, an inner surface, and an outer surface, said first bag being provided with a through hole for introducing said user's genitals, namely penis and scrotum, wherein said device further comprises a second urine-permeable bag, placed inside said first bag, in which a hydrogel-based product adapted to transform said user's urine from the liquid to the solid state is housed. According to embodiments, the product contained in said second bag further comprises perfuming substances or substances which change the organoleptic properties of urine.

According to embodiments, said first bag is made of PVC or a biodegradable material.

According to embodiments, the through hole has an elasticized curled edge to facilitate the introduction of the genitals.

According to embodiments, the device comprises absorbent means for the unsolidified urine.

According to embodiments, the absorbent means comprise at least one absorbent sponge for urine reflux, which is applied at the edge of said hole.

According to embodiments, the absorbent means comprise an alginate-type absorbent coating which covers the inner surface of said first bag.

The device can further comprise retention means for keeping said first bag in place when using the device.

According to embodiments, the retention means comprise a double-sided tape positioned at least at a lower portion of the outer side of said edge of said hole.

According to embodiments, the retention means comprise mesh underpants to be worn over said device to keep said first bag in place when using the device.

According to embodiments, the retention means comprise an elastic harness having two loops for the introduction of the user's legs connected to an adjustable belt at the user's pelvis.

According to embodiments, said first bag externally has, around said hole, an attachment collar for said harness.

According to embodiments, said front and rear walls are connected by means of bellows sidewalls.

According to embodiments, the folds of said bellows sidewalls limit the lateral displacement of said second bag.

### Brief description of the Figures

Further features and advantages of the invention will become more apparent from the description of a preferred but not exclusive embodiment of the urinary incontinence device according to the invention, shown by way of a non-limiting indication in the accompanying drawings, in which:

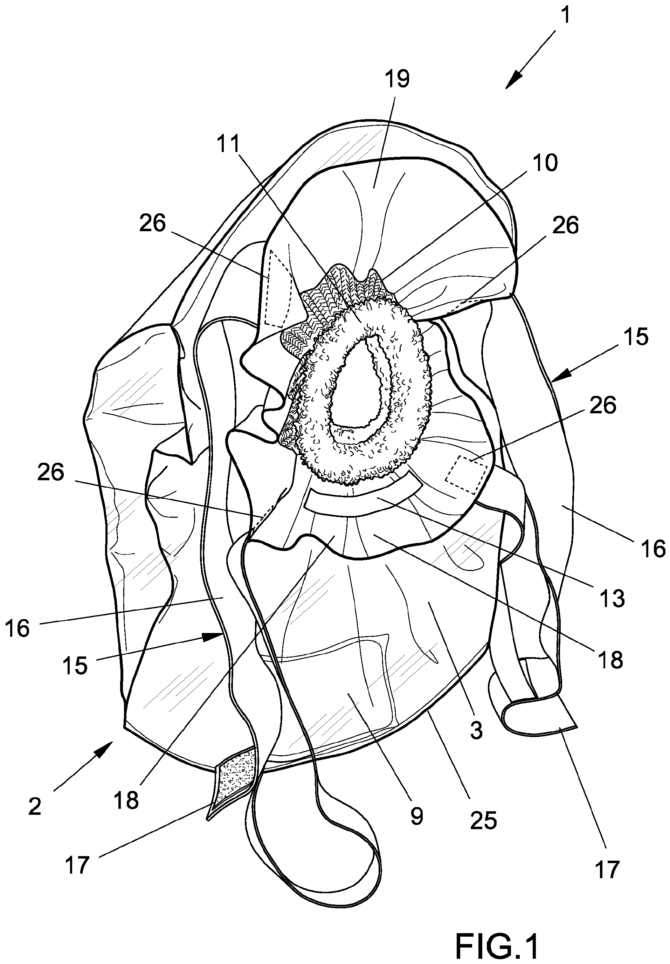

figure 1 shows a diagrammatic plan view of the front wall of the urinary incontinence device according to the present invention;

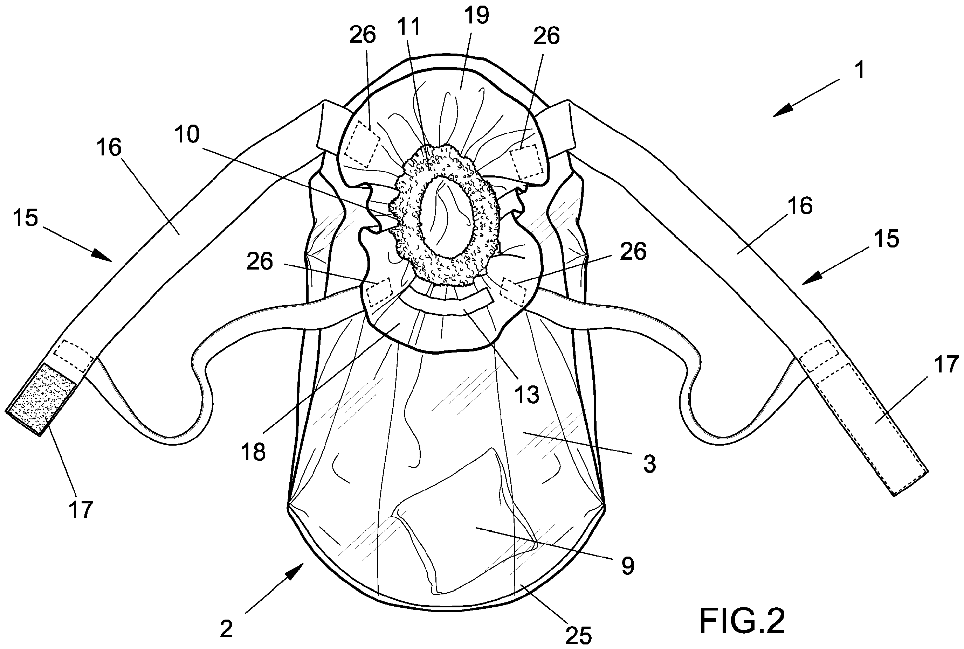

figure 2 shows a diagrammatic plan view of the rear wall of the urinary incontinence device according to the present invention;

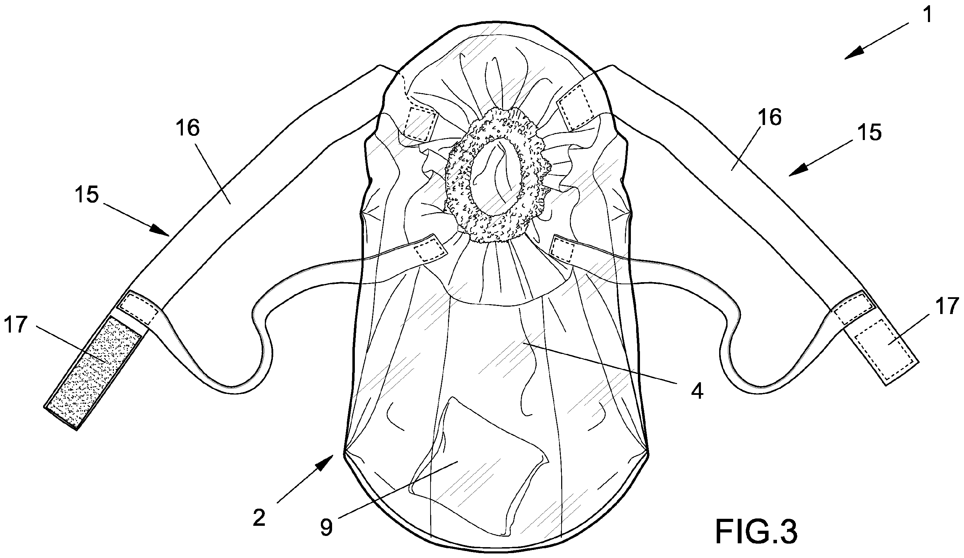

figure 3 shows a perspective view of the urinary incontinence device according to the present invention;

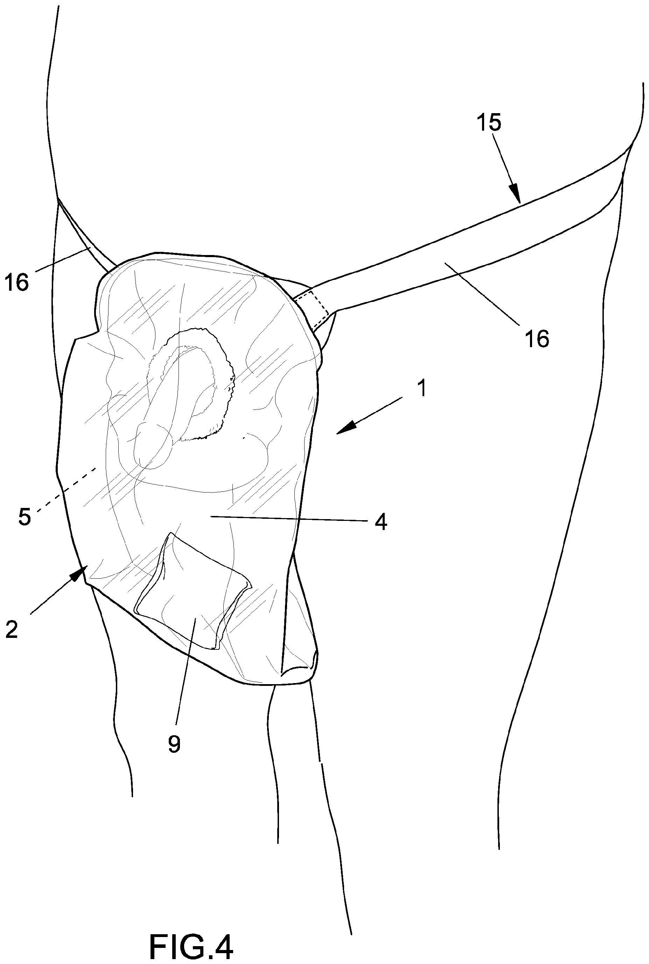

figure 4 shows the device being worn.

### Description of embodiments

With reference to the cited drawings, a male urinary incontinence device is shown, indicated by reference numeral 1 as a whole.

The male urinary incontinence device 1 comprises a first collection bag 2 of a user's urine.

Such a first bag 2 has a rear wall 3, a front wall 4, an inner surface 5, and an outer surface 6.

The first bag 2 also has a bottom 25 where urine is collected. When the device is in use, the rear wall 3 faces and is partially in contact with the user's body, in particular the pubis.

In particular, the first bag 2 is provided with a through hole 7 for the introduction of the genitals, placed in an upper area 8 of the rear wall 3.

Advantageously, the device 1 comprises a second urine-permeable bag 9, placed inside the first bag 2, in which a hydrogel-based product adapted to transform urine from the liquid to the solid state is housed.

In particular, the second bag 9 is positioned at the bottom 25 of said first bag 2 where urine is collected most.

The use of this second bag 9 advantageously allows solving the technical drawback of urine reflux, in particular when the user is lying down.

The first bag 2 can be oval in shape, and can have a length of about 25-30 cm and a width of 15-16 cm.

The product contained in the second bag 9 can also comprise perfuming substances or substances which change the organoleptic properties of urine.

The first bag 2 can be made of polyvinyl chloride (PCV) or a fully biodegradable material.

The through hole 7 can have a size of 6-9 cm and has an elasticized curled edge 10 to facilitate the introduction of the genitals, namely penis and scrotum.

Advantageously, the device 1 comprises absorbent means 11, 12 for the unsolidified urine.

In particular, the absorbent means comprise at least one absorbent sponge 11 for urine reflux, which is applied at the edge 10 of said hole 7.

The absorbent means can further comprise an alginate-type absorbent coating 12 employed to cover the inner surface 5 of the first bag 2.

According to the present invention, means can be provided for improving the portability and safety of the urinary incontinence device.

In fact, the urinary incontinence device comprises retention means adapted to keep the first bag 2 in place when the device is used by the user.

According to an embodiment of the invention, the retention means comprise a double-sided tape 13 positioned at least at a lower portion 18 of the outer side of said edge 10 of said hole 7.

According to another embodiment of the invention, the retention means comprise mesh briefs to be worn over said device 1 to keep the first bag 2 in place when the device is used by the user. Finally, the retention means can comprise an elastic harness 15 having two loops 16 for the introduction of the user's legs, connected to an adjustable belt 17 at the user's pelvis.

Advantageously, the first bag 2 externally has, around the through hole 7, an attachment collar 19 for the harness 15.

The harness 15 is attached to the collar 19 by means of stitches 26. The collar 19 in turn is attached to the edge of the through hole 7 in the interposition of a flap of the sponge 11 by means of stitches.

In particular, each loop 16 is configured as an open ring having the two ends attached to the collar 19 by stitching 26.

The user can thus easily wear the urinary incontinence device by inserting each leg into a corresponding loop 16 of the harness 15, fastening the belt 17 around the pelvis, and then closing it by virtue of closing means present at the ends of said belt, in particular buttons or Velcro.

The first bag 2 further has bellows sidewalls 20 connecting the front wall 4 and the rear wall 3. The bellows sidewalls 20 allow an expansion of the volume of the first bag as it is filled with urine.

Finally, the folds of said bellows sidewalls 20 advantageously limit the lateral displacement of the second bag 9, so that the second bag 9 remains in place at the bottom 25 of said first bag 2 where urine is collected most, even without a mechanical fastening.

This clearly facilitates the absorption of urine by said second bag and the transformation thereof from the liquid to the solid state. The urinary incontinence device 1 according to the present invention is preferably for disposable daily use.

The use mode of the incontinence device 1 according to the present invention is fully apparent from the above description.

The user inserts each leg into a respective loop 16 of the harness 15 connected to the first bag 2.

Before fastening the belt 17 behind the pelvis, the user inserts his genitals into the through hole 7. If the double-sided tape 13 is present, before inserting the genitals, the user removes the film from the tape.

Once the genitals have been inserted into the hole 7, the user adheres the double-sided tape to his pubis and completely fastens the belt 17 around his pelvis so that the bag is well maintained and cannot fall.

For greater stability and safety of the bag, the user can wear the disposable mesh briefs over the positioned device, which allows the first bag to be kept in place.

Once the device is in place, in the case of incontinence episodes, urine is collected at the bottom of the first bag where there is the second bag containing the hydrogel-based product which transforms urine from the liquid to the solid state.

The male urinary incontinence device thus conceived is susceptible to modifications and variations all falling within the scope of protection of the invention.

For example, the first bag 2, unlike what is shown in the accompanying drawings, can have a special inner narrowing which connects a genital containment chamber to a containment chamber of the second bag 9. This inner narrowing, which gives the first bag 2 a vaguely hourglass inner configuration, allows quickly draining urine towards the second bag 9 but prevents or at least limits the annoying ascent of the particles produced by urine solidification towards the genitals.

## Claims

1. A device (1) for male urinary incontinence, comprising a first urine collection bag (2) having a rear wall (3), a front wall (4), an inner surface (5), and an outer surface (6), said first bag (2) being provided with a through hole (7) for introducing the genitals, **characterized in that** said device (1) further comprises a second urine-permeable bag (9), placed inside said first bag (2), in which a hydrogel-based product adapted to transform urine from the liquid to the solid state is housed.

2. The male urinary incontinence device (1) according to claim 1, c wherein said product further comprises perfuming substances or substances which change the organoleptic properties of urine.

3. The male urinary incontinence device (1) according to any of the preceding claim, wherein said first bag (2) is made of PVC.

4. The male urinary incontinence device (1) according to any one of claims 1 and 2, wherein said first bag (2) is made of a biodegradable material.

5. The male urinary incontinence device (1) according to any of the preceding claims, **characterized in that** said through hole (7) has an elasticized curled edge (10) to facilitate the introduction of the genitals.

6. The male urinary incontinence device (1) according to any of the preceding claims, wherein it comprises means (11, 12) for absorbing the unsolidified urine.

7. The male urinary incontinence device (1) according to any of the preceding claims, wherein said absorbent means comprise at least one absorbent sponge (11) for urine reflux, which is applied at the edge (10) of said hole (7).

8. The male urinary incontinence device according to claim 6, wherein said absorbent means comprise an alginate-type absorbent coating (12) which covers the inner surface (5) of said first bag (2).

9. The male urinary incontinence device (1) according to any of the preceding claims, wherein it comprises retention means (13, 14, 15) for keeping said first bag (2) in place when using the device.

10. The male urinary incontinence device (1) according to any of the preceding claims, wherein said retention means comprise a double-sided tape (13) positioned at least at a lower portion (18) of the outer side of said edge (10) of said hole (7).

11. The male urinary incontinence device (1) according to claim 6, wherein said retention means comprise mesh briefs (14) to be worn over said device (1) to keep said first bag (2) in place when using the device (1).

12. The male urinary incontinence device (1) according to any of the preceding claims, wherein said retention means comprise an elastic harness (15) having two loops (16) for the introduction of the user's legs connected to an adjustable belt (17) at the user's pelvis.

13. The male urinary incontinence device (1) according to any of the preceding claims, wherein said first bag (2) externally has, around said hole (7), an attachment collar (19) for said harness (15).

14. The male urinary incontinence device (1) according to any of the preceding claims, wherein said front (4) and rear (3) walls are connected by means of bellows sidewalls (20), the folds of which limit the lateral displacement of said second bag (9).

15. The male urinary incontinence device (1) according to any of the preceding claims, wherein said first bag (2) has an inner narrowing which connects a genital containment chamber to a containment chamber of said second bag (9).

16. The male urinary incontinence device (1) according to any of the preceding claims, wherein the genitals are penis and scrotum.
